# FASCICULE DE BREVET EUROPEEN

(11) **EP 1 287 850 B1**
(45) Date de publication et mention de la délivrance du brevet: **23.01.2013**
(21) Numéro de dépôt: 02292174.6
(22) Date de dépôt: 04.09.2002
(51) Int. Cl.: A61N 1/37, A61N 1/365

(54) **Dispositif médical implantable actif pour le traitement des troubles du rythme cardiaque, comprenant des moyens de détection, d'analyse et de traitement des pauses ventriculaires**
Aktive implantierbare medizinische Vorrichtung zur Behandlung von Herzrhythmusstörungen, mit Mitteln zur Erkennung, Analyse und Behandlung von ventrikulären Pausen
Active implantable medical device for treating heart rhythm disturbances, comprising means for detection, analysis and treatment of ventricular pauses

(30) Priorité: 04.09.2001 FR 0111396
(43) Date de publication de la demande: 05.03.2003
(73) Titulaire: ELA MEDICAL, 92541 Montrouge (FR)
(72) Inventeur: Poezevera, Yann, 91080 Cour couronne (FR)
(74) Mandataire: Dupuis-Latour, Dominique

(56) Documents cités:
- EP-A- 0 472 411
- US-A- 5 261 401
- US-A- 5 306 293

## Description

L'invention concerne les "dispositifs médicaux implantables actifs" tels que définis par la directive 90/385/CEE du 20 juin 1990 du Conseil des communautés européennes, et plus précisément les dispositifs stimulateurs cardiaques, défibrillateurs et/ou cardioverteurs qui sont équipés de fonctions mémoire dites "Holter" permettant un suivi continu des paramètres du rythme cardiaque ainsi que de mémorisation de marqueurs d'événements (tels que stimulation, détection en/hors période réfractaire, capteur, etc.), en particulier pour évaluer la stabilité de celui-ci à des fins diagnostiques et/ou thérapeutiques.

L'invention vise plus particulièrement le cas de certains patients présentant de façon paroxystique les symptômes d'une bradycardie, tels que syncopes inexpliquées, mais sans qu'une bradycardie puisse être réellement observée et documentée par un enregistrement externe temporaire de type Holter.

Pour permettre au praticien de poser un diagnostic et de décider de l'opportunité ou non de l'implantation d'un stimulateur cardiaque, il a été proposé d'implanter un dispositif à visée purement diagnostique permettant d'enregistrer des informations sur le rythme cardiaque afin de confirmer ou d'infirmer la cause rythmique de divers symptômes observés chez le patient (dispositif *Reveal® Insertable Loop Recorder* de Medtronic, Inc.). Toutefois ce dispositif ne comporte aucun moyen de stimulation cardiaque (il est d'ailleurs dépourvu de sonde endocavitaire de manière à simplifier son implantation), de sorte qu'il n'offre aucun secours si surviennent des bradycardies sévères dont les conséquences peuvent être extrêmement délétères chez les patients à risque d'arythmie ventriculaire.

Il serait certes possible d'implanter chez le patient, à titre prophylactique, un stimulateur complet équipé de fonctions mémoire Holter. L'appareil serait alors, dans un premier temps, utilisé seulement pour ses capacités Holter afin d'enregistrer des informations sur le rythme cardiaque au moment des symptômes évoqués par le patient, en vue de confirmer ou infirmer la cause rythmique. Mais pour être en mesure d'enregistrer l'activité cardiaque spontanée du patient, il est bien entendu nécessaire d'inhiber la fonction de stimulation cardiaque du dispositif.

Dans ce cas de figure, il n'est pas opportun d'activer les moyens de stimulation, car l'indication d'une stimulation par l'appareil n'est pas encore démontrée ; cependant, aux yeux du patient, la présence d'un stimulateur et l'acceptation d'une intervention chirurgicale pour l'implantation de celui-ci rend intolérable la persistance de symptômes parfois sévères, et dont les conséquences peuvent être extrêmement graves.

Il serait donc indispensable dans ce cas de prévoir une stimulation ventriculaire de secours.

Mais un stimulateur cardiaque classique ne serait pas adapté : en effet, la fréquence de stimulation de base ne peut pas être programmée à des valeurs suffisamment basses pour laisser s'exprimer le rythme ventriculaire spontané lors d'une bradycardie. Et, quand bien même le stimulateur pourrait disposer de valeurs de fréquence de base assez basses, ces valeurs seraient fixes et arbitraires, et pourraient donc se révéler insuffisantes ou inadaptées au patient.

Tel est le cas du dispositif décrit par le US 5 261 401 A, qui prévoit d'inhiber la stimulation pour permettre la détection d'éventuelles pauses ventriculaires, mais sur la base d'un seuil programmable, donc figé et un évolutif, sauf à reprogrammer le dispositif.

Il n'est en effet pas possible de définir *a priori* la fréquence de stimulation ventriculaire de secours, qui doit être suffisamment basse pour ne pas perturber l'enregistrement de l'activité cardiaque spontanée, et suffisamment élevée pour ne pas laisser s'exprimer une bradycardie trop sévère. Ce compromis est impossible à trouver de façon générale, car le seuil de sévérité d'une bradycardie donnant lieu à des symptômes ressentis est propre à chaque patient.

L'invention a pour but de remédier aux inconvénients précités, en proposant un dispositif apte à rechercher des pauses ventriculaires de façon progressive et adaptée, afin de s'approcher au plus près du seuil symptomatique propre au patient à l'instant considéré, tout en assurant une stimulation de secours dès l'enregistrement effectué.

Le dispositif de l'invention est du type divulgué par le US 5 261 401 A correspondant au préambule de la revendication 1. Les moyens propres à l'invention sont énoncés dans la partie caractérisante de cette revendication.

Les sous-revendications exposent des formes de réalisation subsidiaires avantageuses.

On va maintenant décrire un exemple de mise en oeuvre de l'invention, en référence aux dessins annexés.

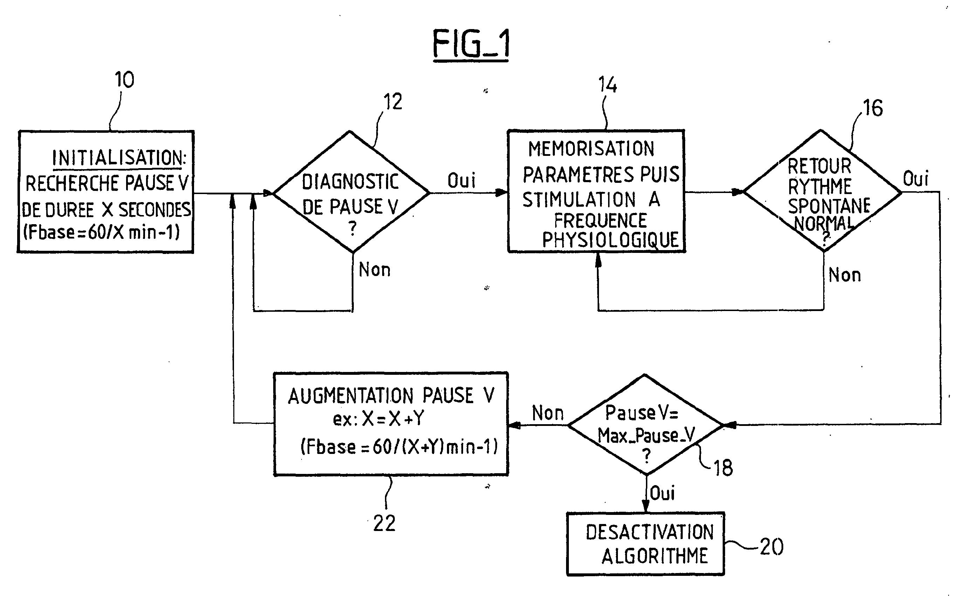

La figure 1 est un organigramme montrant les différentes fonctions mises en oeuvre par le dispositif de l'invention, ainsi que leur enchaînement.

L'invention peut être avantageusement mise en oeuvre par un implant existant tel qu'un stimulateur cardiaque, défibrillateur et/ou cardioverteur piloté par logiciel, les fonctions particulières de l'invention étant mises en oeuvre par une programmation appropriée de ce logiciel.

Ces dispositifs sont, de manière connue, équipés de moyens de stimulation ventriculaire ainsi que de moyens permettant de détecter la présence ou non d'une activité cardiaque ventriculaire spontanée, et, dans l'affirmative, analyser le caractère normal ou non de cette activité spontanée (cycles réguliers et cohérents, fréquence conforme à l'activité du patient, etc.).

Si l'activité spontanée est absente ou anormale, les moyens de stimulation ventriculaire sont commandés de manière appropriée pour appliquer au myocarde des impulsions aptes à provoquer la dépolarisation de celui-ci à des instants prédéfinis, déterminés par le logiciel de pilotage du stimulateur.

Le dispositif est également équipé de fonctions Holter permettant un suivi continu des paramètres du rythme cardiaque ainsi que la mémorisation de marqueurs d'événements. Généralement, s'agissant d'un dispositif implanté, la capacité de la mémoire d'enregistrement est limitée, de sorte que l'on prévoit d'opérer l'enregistrement en boucle, c'est-à-dire que tant que l'enregistrement continue les données les plus récentes viennent remplacer les plus anciennes.

Essentiellement, l'invention consiste à inhiber toute stimulation de manière à laisser s'exprimer le rythme spontané et donc permettre la survenue de pauses ventriculaires. Dès qu'une pause est détectée, les dernières minutes d'enregistrement du rythme cardiaque dans la mémoire du stimulateur sont gelées à des fins d'analyse ultérieure pour que le praticien puisse déterminer la cause, cardiaque ou non, de la pause ventriculaire et décider s'il est opportun ou non d'activer de façon permanente les fonctions de stimulation du dispositif.

Plus précisément, l'algorithme que l'on va décrire et qui est mis en oeuvre dans le dispositif permet de rechercher des pauses ventriculaires de façon progressive, afin de s'approcher au plus près du seuil symptomatique, tout en étant certain de pouvoir appliquer au patient une stimulation de secours chaque fois qu'une pause sera détectée.

Pour ce faire, selon l'invention, le dispositif est doté d'une fréquence de base variable, lentement décroissante au fur et à mesure des pauses ventriculaires détectées.

Dans un premier temps (étape 10), la fréquence de base est initialisée à une valeur qui autorise une pause ventriculaire de durée raisonnable, par exemple une pause de trois secondes (soit une fréquence de base de 20 cpm). Cette valeur très basse, non physiologique (c'est-à-dire située très en deçà d'un rythme spontané normal), laissera s'exprimer d'éventuelles bradycardies. La valeur initiale de 20 cpm, correspondant à une pause de trois secondes, n'est bien entendu donnée qu'à titre d'exemple, mais en pratique elle correspond à une valeur limite de pause ventriculaire non pathologique, par exemple apparaissant de façon normale pendant le sommeil chez des patients sujets à des phénomènes d'apnée.

Le dispositif mesure alors en continu la durée du cycle ventriculaire courant. Si cette durée atteint une valeur correspondant à la fréquence de base, le dispositif diagnostique alors la survenue d'une pause ventriculaire (étape 12) et déclenche alors deux actions (étape 14) :
- sauvegarde des dernières informations Holter enregistrées avant la pause, par exemple sauvegarde des paramètres enregistrés pendant les trois secondes précédant le début de la pause ventriculaire. Les informations Holter enregistrées en continu comportent notamment un électrocardiogramme endocavitaire, ainsi que des marqueurs et diverses données concernant par exemple la ventilation-minute, l'état d'un capteur d'effort, etc.
- délivrance immédiate d'une stimulation à fréquence physiologique, par exemple 50 cpm ou plus (cette valeur pouvant être programmée à l'avance), et ce pendant plusieurs cycles de manière à minimiser ou rendre imperceptible les éventuels symptômes consécutifs à la pause ventriculaire.

La stimulation de secours à fréquence physiologique est maintenue tant qu'un rythme spontané normal n'est pas retrouvé, ce rythme étant par exemple testé après dix cycles de stimulation à fréquence physiologique (étape 16) :
- en l'absence de retour à un rythme spontané normal, ceci signifie que le trouble que l'on croyait fugace est devenu chronique, et le patient a donc alors en tout état de cause besoin d'une stimulation permanente.
- en cas de rétablissement d'un rythme spontané normal :
   si la dernière pause détectée (à l'étape 12) correspond à la valeur maximum de pause fixée par le praticien (étape 18), alors l'algorithme peut être désactivé (étape 20) ; le dispositif gèle alors les informations contenues dans ses mémoires, de sorte qu'il sera ensuite possible de décharger au moyen d'une liaison par radiofréquences les informations de ces mémoires vers un système extérieur en vue d'une analyse par le praticien.
   dans le cas contraire, le dispositif recalcule (étape 22) une nouvelle fréquence de base plus basse, autorisant donc une pause plus longue ; la recherche de pause (étape 12) est alors réitérée sur ces nouvelles bases, la stimulation à fréquence physiologique ayant bien entendu été interrompue dès disparition de la bradycardie (test de l'étape 16).

La réitération de l'algorithme permet de laisser apparaître, et d'enregistrer, des pauses ventriculaires de sévérité croissante, qui finiront par être perçues par le patient. Ce dernier pourra alors se manifester auprès du praticien dès l'apparition de ces symptômes - la stimulation de secours restant active dans l'intervalle.

Le patient tire ainsi profit à la fois des capacités Holter de l'implant (qui permettront le diagnostic ultérieur par le praticien) et de la sécurité procurée par la stimulation de secours, cette dernière n'étant mise en oeuvre que dans les situations où elle s'avère réellement nécessaire.

## Revendications

1. Un dispositif médical implantable actif, notamment un stimulateur cardiaque, défibrillateur et/ou cardioverteur, comprenant :
- des moyens de stimulation ventriculaire,
- des moyens de détection d'une activité cardiaque ventriculaire spontanée,
- des moyens de commande des moyens de stimulation, propres à commander l'application d'une stimulation en l'absence d'activité cardiaque ventriculaire spontanée, et
- des moyens d'enregistrement en continu des événements cardiaques courants ou de paramètres représentatifs de ces événements,
- des moyens d'inhibition contrôlée des moyens de stimulation ventriculaire,
- des moyens (12) de détection de pauses ventriculaires de durée supérieure à une valeur prédéterminée (X), lesdites pauses survenant pendant des périodes d'inhibition contrôlée des moyens de stimulation ventriculaire, et
- des moyens (14) pour sauvegarder les derniers événements cardiaques courants et/ou marqueurs d'événements précédant la détection de la pause ventriculaire,
dispositif **caractérisé en ce qu'**il comprend des moyens (22) aptes, après détection d'une pause ventriculaire par les moyens de détection, à augmenter par pas successifs (Y) ladite valeur prédéterminée (X) des moyens de détection de pauses ventriculaires, de sorte que cette valeur évolue dans le temps de manière progressive.

2. Le dispositif de la revendication 1, dans lequel ladite augmentation est opérée itérativement jusqu'à un seuil maximum programmé (Max_Pause_V).

3. Le dispositif de la revendication 1, du type stimulateur double chambre, comprenant :
- des moyens de détection d'une activité cardiaque auriculaire, et
- des moyens discriminateurs de l'origine de la bradycardie causée par le trouble du rythme cardiaque.

4. Le dispositif de la revendication 1, comprenant en outre :
- des moyens (14) pour commander les moyens de stimulation à une fréquence physiologique de secours en cas de pause ventriculaire détectée.

5. Le dispositif de la revendication 1, dans lequel lesdits moyens pour augmenter ne sont commandés qu'après que les moyens de détection aient préalablement détecté (16) une activité cardiaque ventriculaire spontanée normale.

6. Le dispositif de la revendication 1, comprenant en outre :
- des moyens (20) de désactivation permanente, après sauvegarde des derniers événements cardiaques courants et/ou marqueurs d'événements, desdits moyens d'inhibition contrôlée des moyens de stimulation ventriculaire.

7. Le dispositif de la revendication 6, dans lequel les moyens de désactivation permanente (20) ne sont mis en oeuvre que si lesdits moyens pour augmenter ont opéré ladite augmentation jusqu'audit seuil programmé (18) et après que les moyens de détection aient préalablement détecté (16) une activité cardiaque ventriculaire spontanée normale.

## Claims

1. Active implantable medical device, notably a cardiac stimulator, defibrillator and/or cardioverter, comprising:
- ventricular stimulation means,
- means for detecting a spontaneous ventricular cardiac activity,
- means for controlling the stimulation means, specifically for controlling the application of a stimulation in the absence of spontaneous ventricular cardiac activity, and
- means for continuously recording current cardiac events or parameters representative of these events,
- means for the controlled inhibition of the ventricular stimulation means,
- means (12) for detecting ventricular pauses over a duration greater than a predetermined value (X), said pauses occurring during periods of controlled inhibition of the ventricular stimulation means, and
- means (14) for saving the latest current cardiac events and/or event markers preceding the detection of the ventricular pause,
the device being **characterized in that** it comprises means (22) that are suitable, after the detection of a ventricular pause by the detection means, for increasing, by successive increments (Y), said predetermined value (X) of the means for detecting ventricular pauses, so that this value changes over time in a progressive manner.

2. Device of Claim 1, in which said increase is applied iteratively up to a programmed maximum threshold (Max_Pause_V).

3. Device of Claim 1, of the double chamber stimulator type, comprising:
- means for detecting an auricular cardiac activity, and
- means for discriminating the origin of the bradycardia caused by the perturbation of the cardiac rhythm.

4. Device of Claim 1, also comprising:
- means (14) for controlling the stimulation means at an emergency physiological frequency in the case of a detected ventricular pause.

5. Device of Claim 1, in which said increasing means are controlled only after the detection means have previously detected (16) a normal spontaneous ventricular cardiac activity.

6. Device of Claim 1, also comprising:
- means (20) for permanently deactivating, after saving the latest current cardiac events and/or event markers, said means for the controlled inhibition of the ventricular stimulation means.

7. Device of Claim 6, in which the permanent deactivation means (20) are implemented only if said increasing means have applied said increase up to said programmed threshold (18) and after the detection means have previously detected (16) a normal spontaneous ventricular cardiac activity.

## Patentansprüche

1. Aktive implantierbare medizinische Vorrichtung, insbesondere ein Herzschrittmacher, Defibrillator und/oder Cardioverter, die enthält:
- Einrichtungen zur ventrikulären Stimulation,
- Einrichtungen zur Erfassung einer spontanen ventrikulären Herzaktivität,
- Einrichtungen zur Steuerung der Stimulationseinrichtungen, die die Anwendung einer Stimulation in Abwesenheit einer spontanen ventrikulären Herzaktivität steuern können, und
- Einrichtungen zur kontinuierlichen Speicherung der laufenden Herzereignisse oder von für diese Ereignisse repräsentativen Parametern,
- Einrichtungen zur kontrollierten Inhibition der Einrichtungen zur ventrikulären Stimulation,
- Einrichtungen (12) zur Erfassung von ventrikulären Pausen einer Dauer länger als ein vorbestimmter Wert (X), wobei die Pausen während Perioden kontrollierter Inhibition der Einrichtungen zur ventrikulären Stimulation auftreten, und
- Einrichtungen (14), um die letzten laufenden Herzereignisse und/oder Ereignismarker vor der Erfassung der ventrikulären Pause zu speichern,
wobei die Vorrichtung **dadurch gekennzeichnet ist, dass** sie Einrichtungen (22) enthält, die nach Erfassung einer ventrikulären Pause durch die Erfassungseinrichtungen in aufeinanderfolgenden Schritten (Y) den vorbestimmten Wert (X) der Einrichtungen zur Erfassung ventrikulärer Pausen erhöhen können, so dass dieser Wert sich zeitlich progressiv entwickelt.

2. Vorrichtung nach Anspruch 1, bei der die Erhöhung iterativ bis zu einer programmierten maximalen Schwelle (Max_Pause_V) durchgeführt wird.

3. Vorrichtung nach Anspruch 1, von der Art Doppelkammer-Schrittmacher, die enthält:
- Einrichtungen zur Erfassung einer aurikulären Herzaktivität, und
- Unterscheidungseinrichtungen des Ursprungs der Brachykardie, die von der Herzrhythmusstörung verursacht wurde.

4. Vorrichtung nach Anspruch 1, die außerdem enthält:
- Einrichtungen (14) zum Steuern der Stimulationseinrichtungen mit einer physiologischen Hilfsfrequenz im Fall einer erfassten ventrikulären Pause.

5. Vorrichtung nach Anspruch 1, bei der die Erhöhungseinrichtungen erst gesteuert werden, nachdem die Erfassungseinrichtungen vorher eine normale spontane ventrikuläre Herzaktivität erfasst haben (16).

6. Vorrichtung nach Anspruch 1, die außerdem enthält:
- Einrichtungen (20) zur permanenten Deaktivierung, nach Speicherung der letzten laufenden Herzereignisse und/oder Ereignismarker, der Einrichtungen zur kontrollierten Inhibition der Einrichtungen zur ventrikulären Stimulation.

7. Vorrichtung nach Anspruch 6, bei der die Einrichtungen zur permanenten Deaktivierung (20) nur angewendet werden, wenn die Erhöhungseinrichtungen die Erhöhung bis zur programmierten Schwelle (18) durchgeführt haben, und nachdem die Erfassungseinrichtungen vorher eine normale spontane ventrikuläre Herzaktivität erfasst haben (16).
